# EUROPEAN PATENT APPLICATION

(11) **EP 4 697 343 A1**
(43) Date of publication of application: **18.02.2026**
(21) Application number: 25764537.4
(22) Date of filing: 07.01.2025
(51) Int. Cl.: G16H 20/30, A61B 5/22

(54) **REMOTE MEDICAL TREATMENT ASSISTANCE SYSTEM**

(30) Priority: 28.06.2024 JP 2024105085
(71) Applicant: UT Healthtech Co., Ltd., Fukushima-shi, Fukushima 960-8053 (JP)
(72) Inventor: KURIHARA, Yuya, Fukushima 9608053 (JP); SHIRATO, Osamu, Hokkaido 0640952 (JP); ENDO, Tatsuya, Fukushima 9693553 (JP); ORIBE, Kazuya, Fukushima 9608053 (JP)
(74) Representative: Weihs, Bruno Konrad
(86) International application number: PCT/JP2025/000201
(87) International publication number: WO 2026/004188

(57) **Abstract**

Problem: To provide a rehabilitation program for an orthopedic patient through a remote medical care system using a smart device.

Solution: A recovery evaluation score of the patient is calculated by inputting answers to a plurality of questions in a questionnaire using a health-related QOL evaluation scale from the patient's smart device, or the calculated recovery evaluation score is input into a PC of a medical institution. Based on the calculated or input recovery evaluation score, a rehabilitation index is determined for quantitatively evaluating the patient's recovery state on a numerical scale, and based on the determined rehabilitation index, grade of a rehabilitation program grade suited to the patient's recovery state is selected. The selected rehabilitation program is stored in the storage device of the PC at the medical institution together with the patient's identification number, and by inputting the patient identification number, the rehabilitation program stored in the PC is retrieved and displayed as a video on the screen, enabling the patient to perform the prescribed rehabilitation exercises according to the displayed rehabilitation program.

## Description

### [Technical Field]

The present invention relates to a remote medical care support system for assisting in the selection and execution of rehabilitation programs for orthopedic patients capable of independent exercise.

### [Background Art]

Orthopedic patients with musculoskeletal disorders or disabilities often experience impaired mobility even after completing treatment for their condition. To restore their pre-illness health status, these patients require rehabilitation for a certain period of time. Rehabilitation is conducted with the aim of restoring the patient's mobility following the completion of treatment for their condition.

Rehabilitation guidance for orthopedic patients capable of independent exercise is provided by a physician who examines the patients and prescribes exercise instruction tailored to the patients' conditions as determined through the examination.

Rehabilitation typically takes several months or longer, depending on the type and severity of the treated condition, and the patient's motor function fluctuates during the recovery process. Therefore, during the rehabilitation period, the patient must regularly visit a medical institution for examinations to confirm their current condition and update the rehabilitation program with appropriate loads. As a result, patients are forced to regularly visit hospital for examinations over an extended period, leading to constant crowding of outpatient departments of hospitals.

Recently, a telemedicine system using a computer network system has been proposed. By transmitting videos to patients via a computer network, patients can easily and effectively receive exercise guidance without going to hospital. In the telemedicine system, a questionnaire can be used for examinations. Patients are asked to answer a plurality of questions listed on the questionnaire, and their health status is evaluated based on their responses.

The inventors of the present invention proposed a telemedicine support system that enables physicians or physical therapists to quantitatively evaluate a patient's motor function using a questionnaire, prescribe a rehabilitation program selected based on the quantitative evaluation results, and update the evaluation of the patient's health status as the patient performs the prescribed independent exercises, thereby enabling the rehabilitation program prescribed to the patient to be updated based on the updated evaluation(WO 2023/219056, Patent No. 7344622).

### [Prior Art Documents]

### [Patent Documents]

[Patent Document 1] Patent No. 7344622 (WO 2023/219056)

### [Summary of the invention]

### [Problem to be solved by the invention]

In WO 2023/219056, a dedicated wearable electronic device is connected to a medical institution's system server. However, in today's world where use of smartphones, tablets, and other smart devices has become widespread, utilizing commercially available smart devices is more cost-effective and performs better. In addition, while the system of WO 2023/219056 inputs evaluation scores calculated based on responses to a questionnaire into the system server, it is a significant burden on the medical institution. If patients can accurately recognize their own condition and correctly understand the meaning of each question on the questionnaire, there is no need of intervention by a physician or physical therapist. Instead, the system can be configured so that evaluation scores are calculated based on the patient's own input of responses to each question on the questionnaire, thereby significantly reducing the burden on medical institutions.

### [Means for solving the problem]

Under the above circumstances, the inventors of the present invention have made earnest efforts to solve the above problems and arrived at a remote medical care support system for selecting and executing a rehabilitation program for orthopedic patients capable of independent exercise, wherein
the system comprises a smart device and a PC (personal computer) wirelessly connected to the smart device via an Internet connection,
the PC is installed in a medical institution and accessible by a physician or physical therapist and has an application software of the remote medical care support system installed thereon, the PC is provided with:
   means for receiving input of the patient's identification number, profile, and kind of disease treated,
   means for transmitting and receiving data to and from the smart device,
   a memory device, and
   a CPU,
the smart device has the application software of the remote medical support system installed thereon, the smart device is provided with:
   means for logging into the remote medical care support system by entering the patient's identification number registered in the PC,
   means for receiving input of answers to a plurality of questions in a questionnaire using a health-related quality of life (QOL) assessment scale,
   means for transmitting and receiving data to and from the PC, and
   means for displaying the rehabilitation program received from the PC on an output screen,
the memory device of the PC stores a plurality of rehabilitation programs in video format corresponding to post-operative conditions of specific kinds of diseases,
   each rehabilitation program comprises a plurality of exercise programs,
   each rehabilitation program has a plurality of grades corresponding to a magnitude of load that is placed on a body of the patient when the exercise program is performed,
   each grade of each rehabilitation program has a rehabilitation index registered that is appropriate for the load placed on the body of the patient when the exercise program of that grade is performed,
the system is configured to:
   calculate a recovery evaluation score for the patient based on the patient's responses to the plurality of questions in the questionnaire,
   determine a rehabilitation index that evaluates the patient's recovery status on a numerical scale based on the calculated recovery evaluation score,
   select a rehabilitation program and its grade to be prescribed to the patient from the plurality of rehabilitation programs stored in the memory device of the PC based on the kind of disease of the patient and the rehabilitation index, and
   display the selected rehabilitation program and a grade thereof on an output screen of the smart device.

Preferably, the grade of the rehabilitation program selected based on the rehabilitation index is stored in the memory device of the PC together with the identification number of the patient, and by entering the identification number of the patient registered in the PC from the smart device, the grade of the rehabilitation program stored in the memory device of the PC is retrieved and displayed as a video on an output screen of the smart device.

Preferably, a health management application software is installed in the smart device in addition to the remote medical care support system application software, and the grade of the rehabilitation program selected based on the rehabilitation index is updated based on the patient's exercise biometric information that is measured using the health management application software.

Preferably, the smart device is connected wirelessly or via a wired connection to a wearable sensor device, and the grade of the rehabilitation program selected based on the rehabilitation index is updated based on the patient's exercise biometric information measured using the wearable sensor device.

Preferably, each grade of the rehabilitation program includes exercise biometric information (exercise target value) that is expected to be measured from patients who have performed the exercises instructed in the program of that grade. The exercise target value is set corresponding to the profile of the patient who performs the exercise program. The system is configured to determine whether to change the selected grade of the rehabilitation program by comparing the exercise biometric information measured from the patient who performed the selected grade of the rehabilitation program with the exercise target value determined based on the patient's profile.

Preferably, the predetermined kinds of diseases include musculoskeletal disorders such as low back pain (post-lumbar degenerative disease surgery), scoliosis, post-knee joint surgery, post-hip joint surgery, and chronic low back pain syndrome.

Preferably, the PC further comprises means for receiving input of the patient's recovery evaluation score calculated based on answers to a plurality of questions in a questionnaire using a health-related quality of life (QOL) assessment scale, and is configured to determine the patient's rehabilitation index based on the recovery evaluation score.

The inventors of the present invention further arrived at a remote medical care support system for assisting in the selection and execution of rehabilitation programs for orthopedic patients capable of independent exercise, wherein
the system comprises a smart device and a PC (personal computer) wirelessly connected to the smart device via an internet connection,
the PC is installed in a medical institution and accessible by the patient's attending physician or physical therapist and has the remote medical care support system application software installed thereon, the PC is provided with:
   means for receiving input of patient identification number, profile, and a kind of treated disease,
   means for receiving input of the patient's recovery evaluation score calculated based on answers to a plurality of questions in a questionnaire using a health-related quality of life (QOL) assessment scale,
   means for transmitting and receiving data to and from the smart device,
   a memory device, and
   a CPU,
the smart device has the application software of the remote medical support system installed thereon, the smart device is provided with:
   means for logging into the remote medical support system by entering the patient identification number registered in the PC,
   means for transmitting and receiving data with the PC,
   means for displaying the rehabilitation program received from the PC on an output screen as a video,
the memory device of the PC stores a plurality of rehabilitation programs in video format corresponding to post-operative conditions of specific kinds of diseases,
   each rehabilitation program consists of a plurality of exercise programs,
   each rehabilitation program has a plurality of grades corresponding to the magnitude of the load that is placed on the body of the patient when the exercise programs are executed as instructed in the rehabilitation program,
   each grade of each rehabilitation program has a patient rehabilitation index registered that is appropriate for the load on the body when the exercise programs of that grade are executed,
the system is configured to:
   determine a rehabilitation index that evaluates the patient's recovery status on a numerical scale based on the recovery evaluation score input to the PC,
   select a rehabilitation program and a grade thereof from the plurality of rehabilitation programs stored in the memory device of the PC based on the type of disease of the patient and the rehabilitation index, and stores the rehabilitation program and a grade thereof in the memory device of the PC along with the patient's identification number, and
   by entering the patient's registered identification number into the smart device, the rehabilitation program stored in the memory device of the PC is retrieved and displayed as a video on the display screen of the smart device.

Preferably, the PC further includes means for receiving the identification number of the medical institution that prescribes the rehabilitation program together with the patient's identification number, and is configured such that the patient can log in to the remote medical care support system by entering the patient's identification number registered in the PC and the identification number of the medical institution from the smart device.

Preferably, the smart device is configured such that a password for the patient to log in to the remote medical care support system is set, and the patient can log in to the remote medical care support system by entering the password along with their identification number from the smart device.

Preferably, the smart device is provided with health management application software in addition to the remote medical care support system application software, and is configured such that the grade of the rehabilitation program selected based on the rehabilitation index is updated based on the patient's exercise biometric information measured using the health management application software.

Preferably, the smart device is connected wirelessly or via a wired connection to a wearable sensor device, and the grade of the rehabilitation program selected based on the rehabilitation index is updated based on the patient's exercise biometric information measured using the wearable sensor device.

Preferably, each grade of the rehabilitation program includes exercise biometric information (exercise target values) that is expected to be measured from patients who performed the exercises instructed in the program of that grade, wherein the exercise target values are set corresponding to the profile of the patient who execute the exercise program, and the system is configured to determine whether to change the selected grade of the rehabilitation program by comparing the exercise biometric information measured from the patient executing the selected grade of the rehabilitation program with the exercise target values that is determined based on the patient's profile.

### [Effect of the invention]

In one preferred embodiment of the present invention, the system is configured such that a recovery evaluation score is calculated based on the patient's answers to a plurality of questions on a questionnaire form entered by the patient from their own smart device to determine a rehabilitation index. As a result, there is no need for medical institutions to enter or calculate the patient's recovery evaluation score based on the patient's answers to the questionnaire form, significantly reducing the burden on medical institutions.

A preferred embodiment of the present invention's system allows patients to input their answers to a plurality of questions on a questionnaire into a smart device without visiting a medical institution, enabling the patient's recovery status to be monitored in real-time as rehabilitation progresses.

A preferred embodiment of the present invention's system can be used by installing remote medical care support application software on commercially available mobile phones or tablets, making it extremely convenient for users and cost-effective, thereby offering high commercial practicality.

In one preferred embodiment of the present invention's system, a rehabilitation program/grade can be selected and prescribed to patients based on a recovery evaluation score/rehabilitation index that are calculated and determined according to the patient's profile (gender, age, height, weight, etc.). As a result, it becomes possible to provide a rehabilitation program that is tailored to individual patients' characteristic through the remote medical care support system.

A preferred embodiment of the present invention's system registers the identification number of the rehabilitation program (patient ID) along with the identification number of the medical institution (hospital ID) that prescribes the rehabilitation program to the patient, enabling a plurality of medical institutions to access and utilize the database of the present invention's telemedicine support system, thereby offering high commercial practicality.

In one preferred embodiment of the present invention, the rehabilitation program is displayed on the display screen of the patient's smart device with audio and video, so that the patient can easily and effectively receive rehabilitation exercise instructions from their own smart device.

In one preferred embodiment of the present invention, commercially available smartwatches are used as wearable sensor devices, which are extremely convenient for users and inexpensive, thereby providing high commercial practicality.

A preferred embodiment of the system of the present invention allows a physician or physical therapist to calculate a recovery evaluation score based on a medical interview and input it into a medical institution's PC instead of having the patient input answers to each question of the questionnaire from their smart device. This makes the system highly practical as it can accommodate cases where the patient does not wish to input answers to the questions of questionnaire from their smart device or finds it difficult to do so.

### [Brief Description of the Drawings]

[Fig. 1] Fig. 1 shows a conceptual diagram of the network of one embodiment of the system of the present invention. Smart device 21 of patient 1, smart devices 22 of patient 2 and smart devices 23 of patient 3 are each connected to a medical institution's PC 10. A wearable sensor device 33 is connected to the smart device 23 of patient 3.
[Figure 2] Figure 2 shows a configuration of a PC used in one embodiment of the system of the present invention.
[Figure 3] Figure 3 shows a configuration of a smart device used in one embodiment of the system of the present invention with a wearable sensor device connected thereto.
[Figure 4] Figure 4 shows an example of a display screen of a smart device used in a system according to one embodiment of the present invention.
[Figure 5] Figures 5(A) and (B) show a configuration of a wearable sensor device (smartwatch) connected to a smart device according to one embodiment of the present invention.
[Fig. 6] Fig. 6 shows an example of an SF-36 questionnaire used in a remote medical care support system according to one embodiment of the present invention.
[Fig. 7] Fig. 7 shows an example of an RDQ questionnaire used in a remote medical care support system according to one embodiment of the present invention.
[Fig. 8] Fig. 8 shows an example of a questionnaire used in a remote medical care support system according to one embodiment of the present invention.

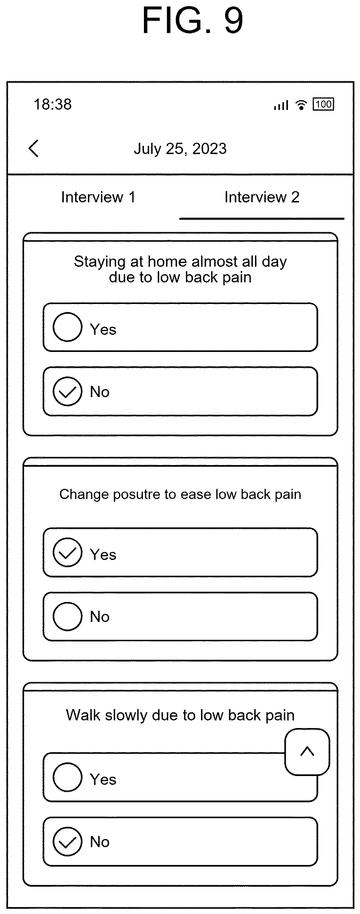

[Figure 9] Figure 9 shows an example of an input screen for answering a plurality of questions in a questionnaire used in a remote medical care support system according to one embodiment of the present invention.
[Figure 10] Figure 10 shows the relationship between recovery evaluation scores, rehabilitation indices, and rehabilitation programs (for patients with low back pain) in a remote medical care support system according to one embodiment of the present invention.
[Figure 11] Figure 11 shows the profile, target heart rate, and calories consumed (for patients with low back pain) of a patient who has undergone one unit of rehabilitation (15 minutes of exercise) in the remote medical care support system of one embodiment of the present invention. Figure 11(A) shows an example for a male patient, and Figure 11(B) shows an example for a female patient.
[Figure 12] Figure 12 shows the relationship between the selection and update of rehabilitation programs (for patients with low back pain) in one embodiment of the remote medical care support system of the present invention.
[Figure 13] Figure 13 shows the relationship between the kind of disease of the patient, the prescribed rehabilitation exercise program, and the rehabilitation index in one embodiment of the remote medical care support system of the present invention.

### [Mode for carrying out the invention]

### Definitions

### <Smart device>

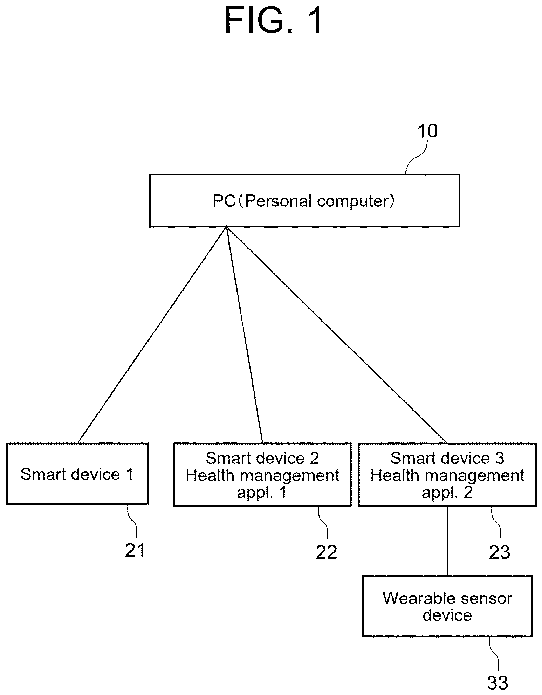

In a preferred embodiment of the present system, a "smart device" refers to a commercially available smartphone (mobile phone) or tablet that is used by installing the application software of the remote medical care support system of the present invention. The smart device is wirelessly connected to a PC at a medical institution via an Internet connection (see Figure 1). In one preferred embodiment of the present invention's system, the smart device is provided with an input unit that allows the patient to log in to the system by entering an identification number (ID) and inputting answers to a plurality of questions of a questionnaire, as well as an output screen that displays rehabilitation programs sent from the medical institution in video format.

### <PC (Personal computer)>

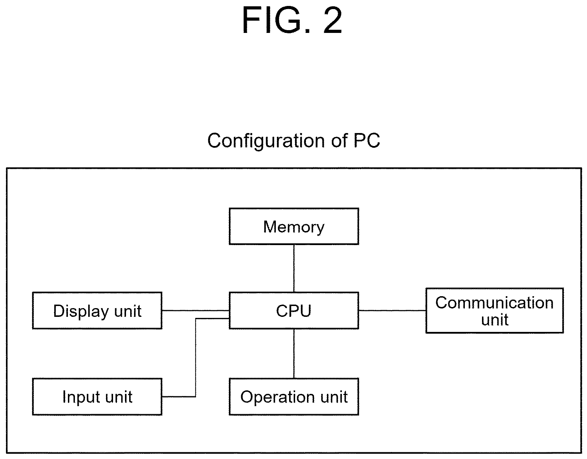

In one preferred embodiment of the present system, a "personal computer (PC)" refers to an apparatus connected to the smart device via an Internet connection and provided with the functionality to install and execute the remote medical care support system of the present invention (see Figure 2). The PC is used with the application software of the remote medical care support system of the present invention installed. The PC's memory device stores a plurality of rehabilitation programs (grades) prescribed for patients, related data, and programs necessary to operate the system of the present invention. The PC includes a CPU (central processing unit), a communication unit for sending and receiving data with smart devices, and an input unit (see Figure 2). The input unit is used to input patient data such as the patient's identification number (ID), profile, and type of disease treated. In another preferred embodiment of the present invention, the PC includes means for accepting input of a recovery evaluation score calculated based on answers to a plurality of questions in a questionnaire.

### <Rehabilitation program>

In one preferred embodiment of the present invention, a "rehabilitation program" refers to an exercise instruction program prescribed for the purpose of restoring the motor function of a patient who completed treatment for a disease, and is configured to instruct a patient to perform a plurality of kinds of exercises in video form.

The rehabilitation program has a plurality of grades, and a grade of the rehabilitation program selected according to the patient's profile (age, gender, weight, height, etc.) and the recovery status of their motor function is prescribed. In one preferred embodiment of the present system, the rehabilitation program is composed of 15-minute units.

### < Wearable sensor device >

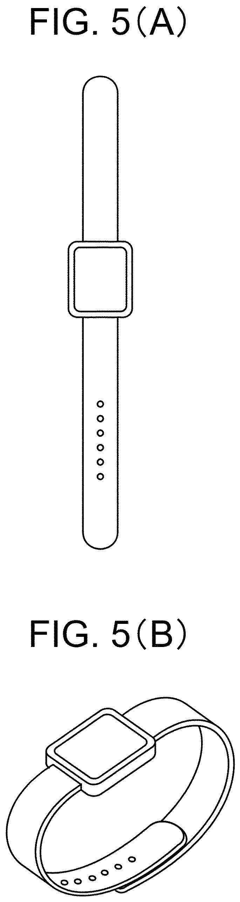

In one preferred embodiment of the present system, a "wearable sensor device" refers to a device that is wirelessly or wired connected to a smart device, measures the patient's exercise biometric information when the patient performs the exercise instructed in the rehabilitation program, and transmits the patient's exercise biometric information to the smart device (see Figures 5(A) and 5(B)). As a wearable sensor device, commercially available smartwatches (Apple Watch, HUAWEI WATCH, etc.) can be used.

### < Remote medical care support system>

In one preferred embodiment of the present system, a "remote medical care support system" refers to a system that can remotely support the selection, execution, or updating of rehabilitation programs and their grades in accordance with the recovery status of a patient's motor functions. The system includes a PC installed at a medical institution and smart device(s) connected to the PC via an internet connection. In one preferred embodiment of the present invention, by connecting a wearable sensor device to a smart device, it becomes possible to measure a patient's exercise biometric information such as heart rate.

### < Health management application software>

In one preferred embodiment of the present invention, a "health management application software" refers to application software installed on a smart device and used for measuring or monitoring a patient's exercise biometric information. Health management application software includes home-use electrocardiogram programs and home-use heart rate monitor programs. Commercially available health management application software such as Google Fit (registered trademark) and Apple Health (registered trademark) may be used.

### <Independent exercise>

In the present invention, "independent exercise" refers to an exercise that a patient can perform on their own under the guidance of a physician or physical therapist, including muscle strength training, muscle endurance training, cardiopulmonary capacity training (whole-body endurance training), flexibility training, dexterity improvement training, coordination improvement training, and running ability improvement training. Walking exercises correspond to muscle strengthening and cardiorespiratory capacity improvement exercise. Sit-to-stand exercise corresponds to muscle strengthening and muscle endurance endurance exercises.

### < Health-related QOL assessment scale >

In a preferred embodiment of the present system, "health-related QOL" refers to a concept that quantifies the impact of diseases and treatments on a patient's subjective sense of health (mental health, vitality, pain, etc.) and on daily activities such as work, housework, and social activities. "Health-related QOL evaluation scales" refer to scales used to objectively evaluate health-related QOL, including disease-nonspecific scales (e.g., SF-36) and disease-specific scales (e.g., RDQ).In a preferred embodiment of the present invention, questions cprepared in accordance with health-related QOL assessment scales are used in the questionnaire items, and the patient's recovery evaluation score is calculated based on the total score assigned to the responses to those questions.

In one preferred embodiment of the system of the present invention, the questions prepared in accordance with health-related QOL scales include questions prepared in accordance with disease-nonspecific scales (e.g., SF-36) that are not related to the specific disease of the patient, and questions prepared in accordance with disease-specific scales (e.g., RDQ, JOABPEQ) that are related to the specific disease of the patient. In the questionnaire used in one preferred embodiment of the present invention, questions based on disease-nonspecific scales and questions based on disease-specific scales are combined to create questionnaire items based on health-related QOL evaluation scales, which are used to evaluate the degree of recovery of the patient's motor function.

In one preferred embodiment of the present invention, SF-36, RDQ, and JOABPEQ are used as health-related QOL scales. SF-36 is a disease-nonspecific scale used to quantitatively evaluate health-related QOL (HRQOL: Health Related Quality of Life).RDQ (Roland Morris Disability Questionnaire) is a scale used by patients to quantitatively evaluate disabilities in daily living caused by low back pain. JOABPEQ (Japanese Orthopedic Association Low Back Pain Questionnaire) is a scale used to calculate severity scores for five categories: pain-related disability, lumbar spine dysfunction, gait dysfunction, social dysfunction, and psychological dysfunction. These scales consist of numerous questions, and the patient's condition is quantitatively evaluated based on the total score assigned to each question according to the patient's responses. The quantitative evaluation of the patient's health status based on these scales is internationally recognized as highly reliable.

### <Recovery evaluation score>

In a preferred embodiment of the present system, a "recovery evaluation score" refers to the total of the scores assigned to each of the patient's responses to a plurality of questions in the questionnaire used in the present invention, and the patient's recovery status of motor function is evaluated based on the total score. In the present invention, the recovery evaluation score is used as a basis for calculating the rehabilitation index. In a preferred embodiment of the present invention, the questionnaire includes non-disease-specific questions and/or disease-specific questions, and based on the patient's responses to these questions, the patient's recovery status of motor function is evaluated in the form of a quantitative score. By including questions related to the patient's profile in the questionnaire items, the recovery evaluation score can be calculated as a value that takes into account the patient's gender, age, height, weight, etc. In this case, the patient's profile is considered in both the initial selection of the rehabilitation program grade and the update of the selected rehabilitation program grade based on the patient's motor biometric information.

### < Disease-non-specific score >

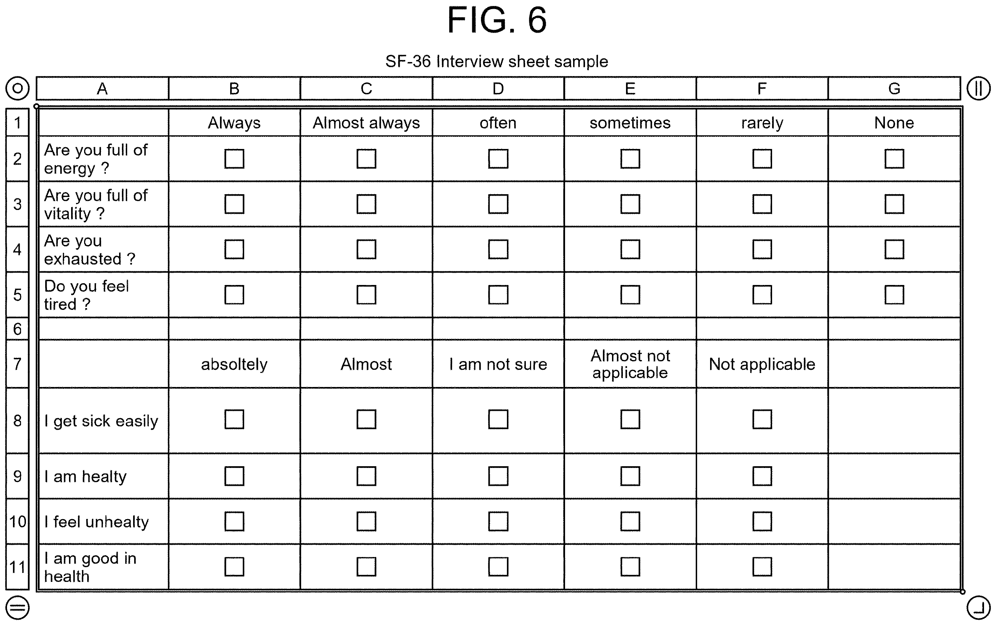

In one preferred embodiment of the present system, a "disease-non-specific score" is a score calculated as the sum of scores assigned to responses to a plurality of questions based on a health-related QOL assessment scale, regardless of the specific disease the patient has.The SF-36 is the most widely used self-report health status questionnaire in the world, designed to measure comprehensive health concepts rather than health status specific to particular diseases or symptoms, through eight domains (see Figure 6).

### < Disease-specific score >

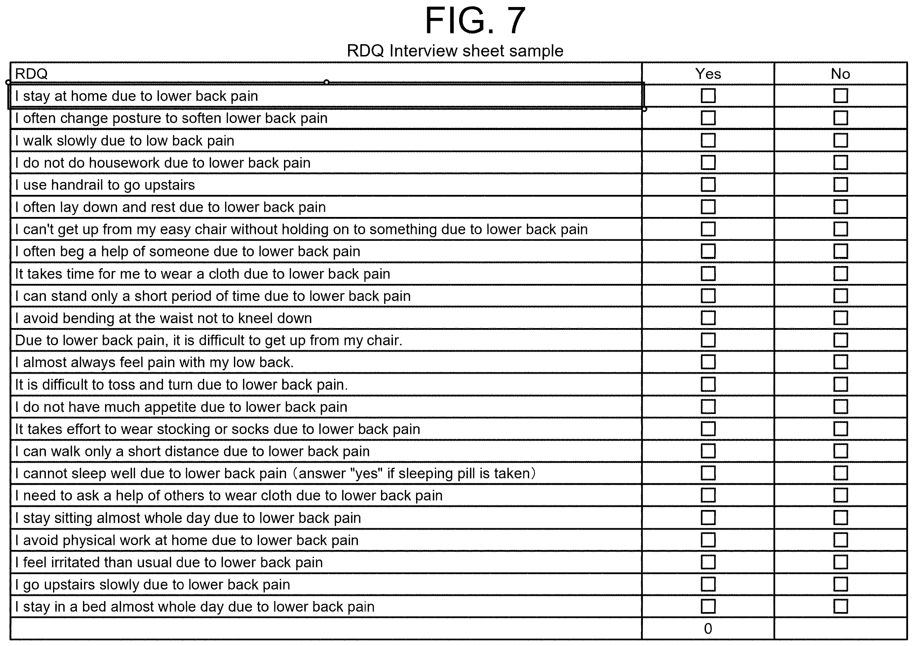

In one preferred embodiment of the present system, a "disease-specific score" is a score calculated in relation to a specific disease that a patient has, and is calculated as the sum of scores assigned to responses to a plurality of questions based on a disease-specific QOL scale. RDQ (Roland Morris Disability Questionnaire) is a set of questions used by patients to quantitatively assess the impairment of daily living due to low back pain (see Figure 7), and JOABPEQ (Japanese Orthopedic Association Low Back Pain Questionnaire) is a set of questions used to calculate five severity scores for pain-related impairment, lumbar spine dysfunction, gait dysfunction, social dysfunction, and psychological dysfunction.

### <Rehabilitation index>

In one preferred embodiment of the present invention, a "rehabilitation index" refers to a value that evaluates the recovery status of a patient's motor function on a numerical scale based on the recovery evaluation score. In a preferred embodiment of the present invention, rehabilitation index is registered in the memory device of the PC together with the exercise target values for each grade of the rehabilitation program.

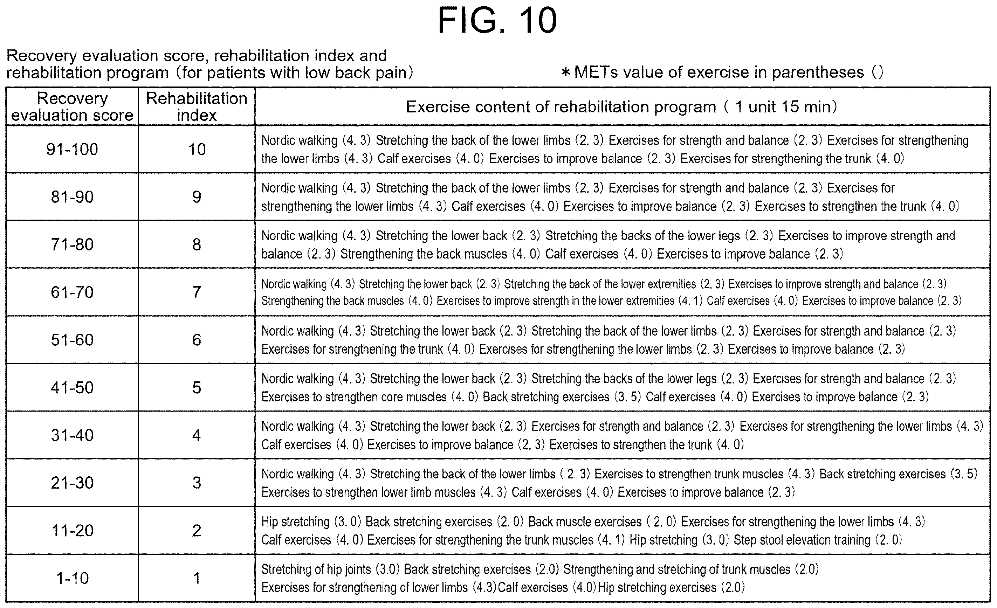

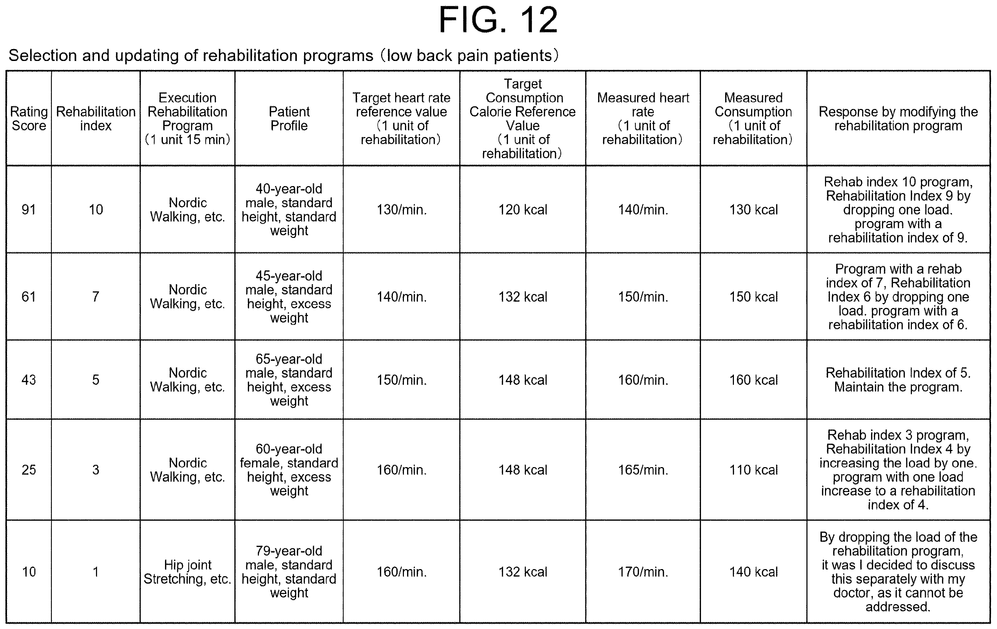

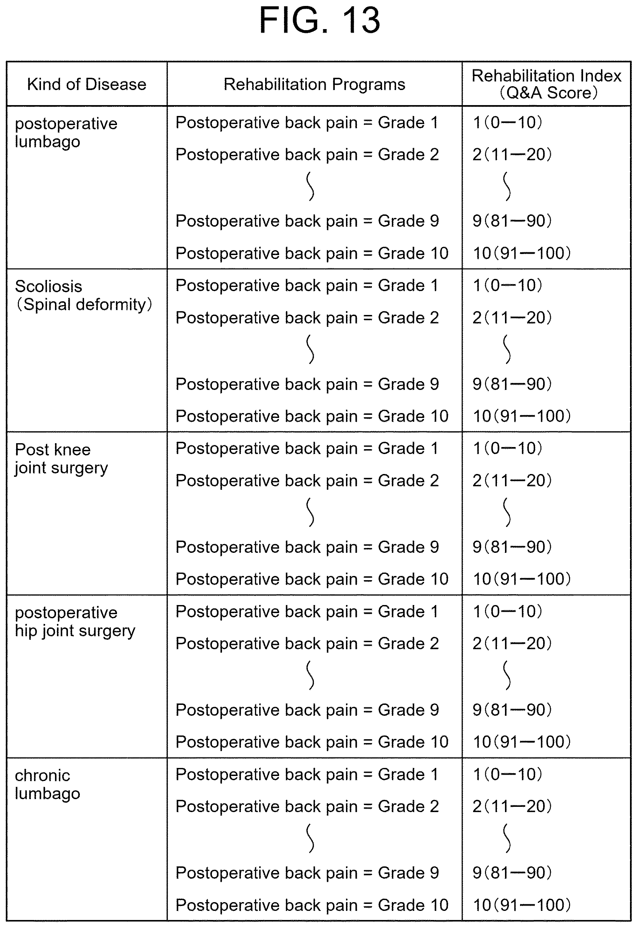

In one preferred embodiment of the present system, when the SF-36 questionnaire is used, the evaluation scores from 1 to 100 obtained from the SF questionnaire are divided into ten levels: 1 to 10 corresponds to Rehabilitation Index 1, 11 to 20 corresponds to Rehabilitation Index 2, 21 to 30 corresponds to Rehabilitation Index 3, 31 to 40 corresponds to Rehabilitation Index 4, 41 to 50 corresponds to Rehabilitation Index 5,51-60 is Rehabilitation Index 6, 61-70 is Rehabilitation Index 7, 71-80 is Rehabilitation Index 8, 81-90 is Rehabilitation Index 9, and 91-100 is Rehabilitation Index 10 (see Figures 10 and 13).For example, if the total score from an SF-36 questionnaire assigned to a patient is 43, the patient's rehabilitation index is 5. When using both disease-nonspecific scores (SF-36) and disease-specific scores (RMDQ) in the questionnaire, the average value obtained by summing the SF-36 evaluation score and the RMDQ evaluation score and dividing by 2 is 25, the patient's rehabilitation index is 3 (see Figure 12).

In cases of low back pain and other conditions, in addition to disease-specific factors with clear causes, disease-nonspecific factors with unclear causes often play a significant role. While RDQ serves as an evaluation criterion related to the patient's low back condition in selecting a rehabilitation program for low back pain, since low back pain may be caused by factors other than low back conditions, it is necessary to evaluate such cases using disease-nonspecific scales. In the present invention, orthopedic patients are interviewed using both disease-specific scale-based questionnaire items and disease-non-specific scale-based questionnaire items, and a rehabilitation index is calculated based on the results of both sets of questionnaire items. Based on the calculated rehabilitation index, a rehabilitation program is selected for patients who have undergone treatment for a specific disease.

In one preferred embodiment of the present invention's system, in addition to questionnaire scores using SF-36, RMDQ, JOABPEQ, etc., a physician or physical therapist can calculate a recovery evaluation score by considering other characteristics or profiles of the patient that are not evaluated by such questionnaires, and finally determine the rehabilitation index.

### < Exercise biometric Information>

In one preferred embodiment of the present system, "exercise biometric information" refers to data measured and collected from patients who have performed exercises instructed in the rehabilitation program. In one embodiment of the present invention, exercise biometric information is measured and collected from the patient's body using a smart device and/or wearable sensor device provided with health management application software. The exercise biometric information may include measurement values that correlate with the magnitude of exercise load, and may include all or part of the following data: calories burned, heart rate, walking distance, walking speed, and number of steps.

### < Exercise target value >

In one preferred embodiment of the present system, a "exercise target value" refer to a target value of exercise biometric information that is expected to be measured from the body of a patient who has performed exercises instructed in a rehabilitation program, and is registered in the memory device of a PC corresponding to rehabilitation indices for each grade of the rehabilitation program. Exercise target values are set for each measurement item according to the patient's profile. In one preferred embodiment of the present invention, exercise target values are determined based on the rehabilitation index of the patient executing the rehabilitation program and the patient's profile input from the input unit, and the exercise target values thus determined are compared with the exercise biometric information measured and collected from the patient's body.

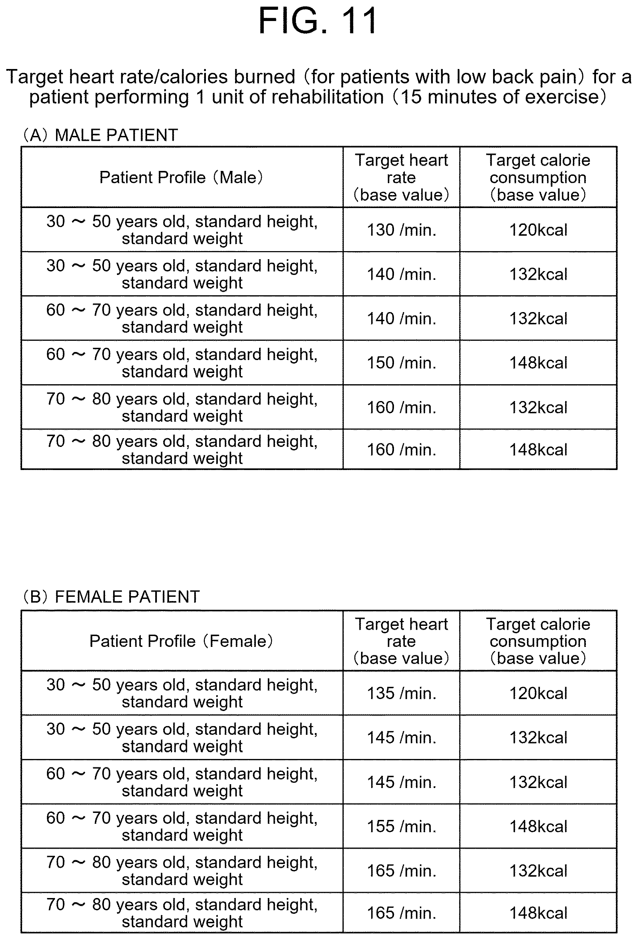

In one preferred embodiment of the present invention, it is determined whether the exercise biometric information measured from the patient for a specific measurement item is within ± 10% of the exercise target value. In this case, the range of percentage above or below the exercise target value is determined by a clinical physician or physical therapist at the medical institution. The exercise target values (target heart rate, target calorie consumption, target walking distance, target number of steps, target walking speed) are not the same for all patients performing the same exercise, and even when performing the same exercise, they vary depending on the individual patient's profile (age, gender, weight, height), so they are set according to the patient's profile. In one preferred embodiment of the present invention, the correspondence between the rehabilitation program, the patient's profile, and the exercise target values is stored in the memory device of the PC, and is used to calculate the exercise target values for each patient who performs the exercises of the rehabilitation program. Figure 11 shows an example of the correspondence between the patient's profile and the target heart rate and target calorie consumption in a rehabilitation program for patients with low back pain.

### < Target calorie consumption>

In one preferred embodiment of the present invention, "target calorie consumption" refers to one of the exercise target values, specifically the number of calories expected to be consumed by a patient who performs the exercise instructed in the rehabilitation program. In a preferred embodiment of the present invention, it is determined whether the patient's measured calorie consumption falls within a range of ± 10% of the registered target calorie consumption value. The amount of energy consumed in an exercise of one unit is determined by the patient's physical condition (body weight), exercise intensity, and exercise duration. The amount of energy (calories) consumed by a patient when performing the prescribed exercise varies depending on the patient's weight, etc., so in the present invention, the target calorie consumption is set according to the patient's profile. In a preferred embodiment of the present invention, if the measured value of calories consumed, as measured and collected by the smart device of a patient who has performed the exercise instructed in the prescribed rehabilitation program, exceeds or falls below a predetermined range above or below the target calorie consumption, the exercise is determined to be too strenuous/too light for the patient.

### < Exercise intensity, METs value >

In one preferred embodiment of the present system, "exercise intensity" refers to the load or difficulty of the exercise performed by the patient as instructed in the rehabilitation program. "METs value" refers to a unit indicating exercise intensity based on the ratio of energy consumed during the exercise to the oxygen consumption at rest (3.5 ml/kg), with the latter set as 1.For example, watching TV while sitting is 1.9 METs, sitting and talking, eating, or desk work is 1.5 METs, and walking around the house is 2.0 METs. In one preferred embodiment of the present invention's system, the exercises in the rehabilitation program include Nordic walking (4.3 METs), stretching the back of the lower limbs (2.3 METs), exercises for muscle strength and balance (2.3 METs),exercises for strengthening lower limb muscles (4.3 METs), calf exercises (4.0 METs), exercises for improving balance (2.3 METs), exercises for strengthening core muscles (4.0 METs), and exercises for strengthening core muscle strength (4.1 METs).One preferred embodiment of the present invention's system enables the calculation of calorie consumption associated with the execution of prescribed rehabilitation program exercises by registering the MET values of each exercise in the rehabilitation programs stored in the PC.

### <Target heart rate>

In one preferred embodiment of the present system, "target heart rate" refers to one of the exercise target values, specifically the heart rate expected to be exhibited by a patient who performs the exercise indicated in the rehabilitation program. Generally, a patient's heart rate increases with greater exercise load and decreases with lesser load. The load imposed on a patient by performing the prescribed exercise varies depending on the patient's gender, age, height, weight, etc. Therefore, in the present invention, the target heart rate is set according to the patient's gender, age, height, weight, etc. In a preferred embodiment of the present invention, if the measured heart rate of a patient who has performed the walking exercise prescribed in the prescribed rehabilitation program exceeds the target heart rate, the exercise is determined to be too strenuous for the patient.

### < Target walking distance >

In a preferred embodiment of the present invention, the "target walking distance" is one of the exercise target values and refers to the distance that a patient is expected to walk within a predetermined time when performing the walking exercise indicated in the rehabilitation program. In the present invention, if the measured walking distance of a patient who has performed the walking exercise indicated in the prescribed rehabilitation program is less than the target walking distance, the exercise is determined to be too strenuous for the patient.

### < Target walking speed >

In one preferred embodiment of the present invention, "target walking speed" refers to one of the exercise target values, specifically the walking speed expected when a patient performs the walking exercise instructed in the rehabilitation program within a specified time. In the present invention, if the measured walking speed of a patient who has performed the walking exercise instructed in the prescribed rehabilitation program falls below or exceeds the target walking speed by a predetermined range, the exercise is determined to be too strenuous or too easy for the patient.

### [Preferred Embodiment of the Invention]

Figure 1 shows the network configuration of a system according to one embodiment of the present invention. A personal computer (PC) 10 operated by a physician in charge is connected via an Internet line to smart devices 21 (smart device 1), 22 (smart device 2), and 23 (smart device 3) carried by patients 1, 2, and 3, respectively. The smart devices 21, 22, and 23 each have the application software of the remote medical support system of the present invention installed. The smart device 22 has health management application software 1 installed. The smart device 23 has health management application software 2 installed and is connected to a wearable sensor device 33.

Referring to Figure 1, the remote medical care support system application software of the present invention is installed on smart device 21. Patient 1 (smart device 1) inputs answers to a plurality of questions on a questionnaire from smart device 21. Based on the recovery evaluation score/rehabilitation index calculated therefrom, the grade of a rehabilitation program with an appropriate load for patient 1 is selected from among a plurality of rehabilitation programs registered in the memory device of PC 10.

The selected rehabilitation program is transmitted from PC 10 to smart device 21 and displayed as a video on the output screen of smart device 21, thereby prescribing exercise instructions to patient 1.

Referring further to Figure 1, smart device 22 has installed, in addition to the application software of the remote medical care support system of the present invention, health management application software 1. Patient 2 (smart device 2) enters answers to a plurality of questions on the questionnaire from smart device 22, and based on the recovery evaluation score/rehabilitation index calculated therefrom, a grade of rehabilitation program with an appropriate load for patient 2 is selected from among a plurality of rehabilitation programs registered in the memory device of PC 10.The selected rehabilitation program is transmitted from PC 10 to smart device 22 and displayed as a video on the output screen of smart device 22, thereby prescribing exercise instructions to patient 2. Smart device 22 has health management application software 1 installed. Using health management application software 1, the exercise biometric information (e.g., number of steps, walking distance) of patient 2 who has performed the rehabilitation exercise is measured. And based on the measured values, the grade of the prescribed rehabilitation program is updated.

Referring further to Figure 1, smart device 23 has the application software of the remote medical care support system of the present invention and health management application software 2 installed. Smart device 23 is further connected to wearable sensor device 33. Patient 3 (smart device 3) inputs answers to a plurality of questions on a questionnaire from smart device 23. Based on the recovery evaluation score/rehabilitation index calculated from these answers, the grade of a rehabilitation program with an appropriate load for patient 3 is selected from a plurality of rehabilitation programs registered in the memory device of PC 10.The selected rehabilitation program is transmitted from PC 10 to smart device 23 and displayed as a video on the output screen of smart device 23, and patient 3 performs the displayed exercise program. Smart device 23 measures the patient's exercise biometric information (e.g., heart rate) using wearable sensor device 33 and compares the measured values with the exercise target values registered for the prescribed rehabilitation program grade to determine whether to update the prescribed rehabilitation program grade. The updated grade of the rehabilitation program is displayed on the output screen of smart device 23 for subsequent rehabilitation exercise guidance.

In another embodiment of Figure 1, physician or physical therapist of the patient can input the recovery evaluation score calculated based on the answers to a plurality of questions in the questionnaire into the input section of PC 10 in the medical institution. In this case, the rehabilitation indices for patients 1, 2, and 3 are determined based on the recovery evaluation scores entered into PC 10 by the physician or physical therapist, and the rehabilitation programs and their grades prescribed for patients 1, 2, and 3 are determined based on the rehabilitation indices thus determined.

Figure 2 illustrates the configuration of the PC (personal computer) used in the present invention. The CPU of the PC processes data received from the smart device via the communication unit and outputs the results to the display unit.

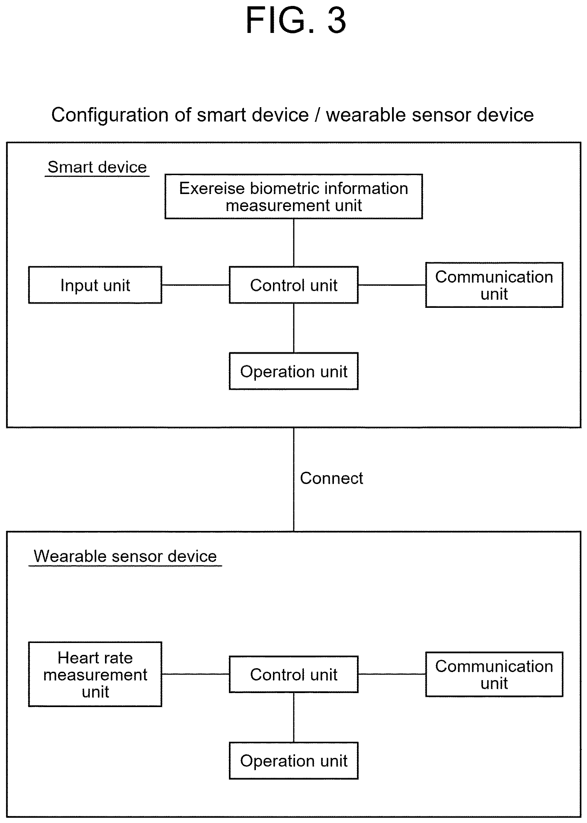

Figure 3 illustrates the configuration of the smart device and wearable sensor device used in one embodiment of the present invention. The smart device includes an input unit for entering responses to questions in the questionnaire, exercise biometric information measurement and collection unit for measuring and collecting the patient's exercise biometric information, and a communication unit for transmitting the measured exercise biometric information to the PC. The smart device shown in Figure 3 (corresponding to smart device 23 in Figure 1) is connected to the wearable sensor device (corresponding to wearable sensor device 33 in Figure 1) via wireless or wired communication.

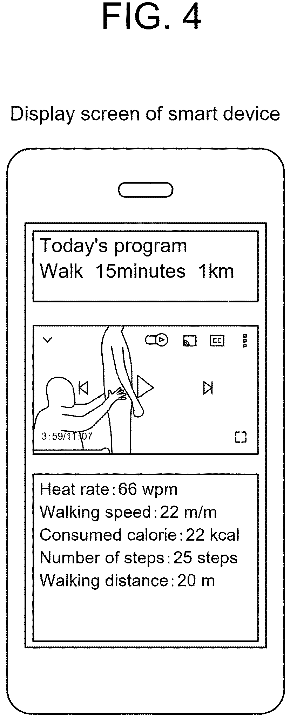

Figure 4 shows an example of an output screen of a smart device used in one embodiment of the present invention. The output screen displays rehabilitation exercises (including videos) to be instructed to the patient along with the patient's exercise biometric information.

Figure 5(A) and (B) show the configuration of a wearable sensor device (smartwatch) used in one preferred embodiment of the present invention. This embodiment is configured to measure the patient's heart rate while the belt is wrapped around the patient's arm.

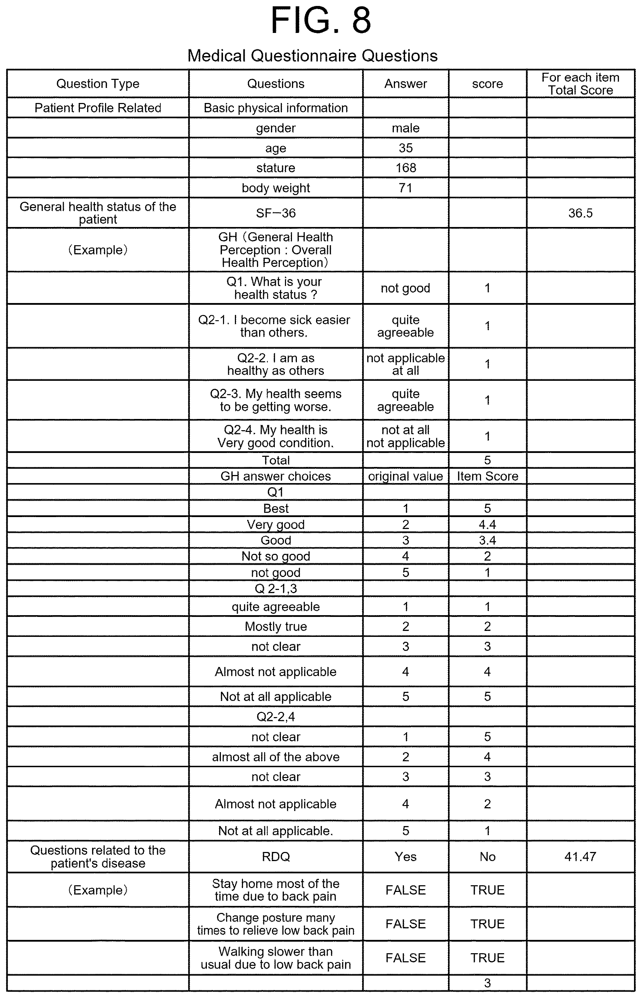

Figure 6 shows a sample of the SF-36 questionnaire used in one embodiment of the present invention, and Figure 7 shows a sample of the RDQ questionnaire used in one embodiment of the present invention. Figure 8 shows a sample of the questionnaire items used in one embodiment of the present invention. The first questions in the questionnaire items relate to the patient's profile (gender, age, height, weight).

### [Example]

Hereinafter, embodiments of the present invention will be explained with reference to the drawings.

### Example 1

### (1) Selection of rehabilitation program

A 65-year-old male visited the clinic due to lower back pain. At the outpatient visit, his height was 170 cm and weight was 85 kg. The SF-36 score was PCS 48, the RDQ score was 38, and the evaluation score obtained by dividing the sum of the two by 2 was 43. Based on the questionnaire results, the rehabilitation index was determined to be 5.As a result, a rehabilitation program (15 minutes per session) including Nordic walking, lower back stretches, hamstring stretches, exercises for muscle strength and balance, core muscle strengthening exercises, back extension exercises, calf exercises, and balance improvement exercises was selected (see Figure 10).

### (2) Update of the rehabilitation program

The selected rehabilitation program was displayed as a video on the output screen of a smart device to instruct the patient to performe the exercises, and heart rate and calorie consumption were measured at the end of each exercise. Based on the patient's height (average) and weight (excess), the target heart rate was set as 150 beats per minute, and the target calorie consumption was set as 148 kcal (refer to Figures 11 and 12).

The patient's heart rate at the end of the rehabilitation exercise was 160 beats per minute, and the calories burned were 160 kcal. Since both the measured heart rate of 160 beats per minute and the calories burned of 160 kcal were within ± 10% of the target heart rate (150% per minute) and target calories burned (148 kcal) set for the prescribed rehabilitation program grade, it was decided to maintain the rehabilitation program grade at 5(See Figure 12).

### Example 2

### Selection of rehabilitation program

A 60-year-old woman visited the clinic due to lower back pain. At the outpatient visit, her height was 150 cm and weight was 60 kg. The SF-36 score was PCS 26, RDQ score was 24, and the evaluation score obtained by dividing the sum of the two by 2 was 25. Based on the results of the medical interview, the rehabilitation index was determined to be 3. As a result, a rehabilitation program (15 minutes per session) including Nordic walking, stretching of the back of the lower limbs, exercises to strengthen core muscles, back-stretching exercises, exercises to strengthen lower limb muscles, calf exercises, and exercises to improve balance was selected (see Figure 10).

### (2) Update of the rehabilitation program

The selected rehabilitation program was displayed as a video on the smart device's screen to instruct the patient to perform the exercises, and heart rate and calorie consumption were measured at the end of each exercise.

Based on the height (standard) and weight (excess) of the female patient who performed the rehabilitation program, the target heart rate was set at 155 beats per minute, and the target calorie consumption was set at 148 kcal (refer to Figures 11 and 12). The patient's heart rate at the end of the rehabilitation exercise was 165 beats per minute, and the calorie consumption was 110 kcal. Since the heart rate (165 beats per minute) was within ± 10% of the target heart rate (160 beats per minute) and the calorie consumption (110 kcal) was below the lower limit of ± 10% of the target calorie consumption (148 kcal), the prescribed program was deemed appropriate for the patient, and it was decided to transition to a higher-grade rehabilitation index 4 program (see Figure 12).

### Example 3

### (1) Selection of rehabilitation program

A 45-year-old male visited the clinic due to lower back pain. At the outpatient visit, his height was 160 cm and weight was 75 kg. The SF-36 score was 60, and the RDQ score was 62. The evaluation score, obtained by summing the two scores and dividing by 2, was 61. Based on the interview results, the rehabilitation index was determined to be 7. As a result, a program including Nordic walking, lower back stretches, stretches for the back of the lower limbs, exercises for muscle strength and balance, exercises for strengthening the back muscles, exercises for strengthening the lower limb muscles, calf exercises, and exercises for improving balance was selected (see Figure 10).

### (2) Update of the rehabilitation program

The selected rehabilitation program was displayed as a video on the smart device's screen to instruct the patient to perform the exercises, and heart rate and calorie consumption were measured at the end of each exercise. Based on the patient's height (standard) and weight (excess), the target heart rate for the selected rehabilitation program was set at 140 beats per minute, and the target calorie consumption was set at 132 kcal (see Figures 11 and 12).The patient's heart rate at the end of the rehabilitation exercise was 150 beats per minute, and the calories burned were 150 kcal. While the measured heart rate of 150 beats per minute was within the ± 10% range of the target heart rate, the calories burned of 150 kcal exceeded the upper limit of the ± 10% range of the target calorie consumption of 132 kcal. Therefore, it was decided to lower the program grade by one level and change to a rehabilitation index 6 program (See Figure 12).

### Example 4

### (1) Selection of rehabilitation program

A 60-year-old woman visited the clinic due to osteoarthritis of the knee. At the outpatient visit, her height was 156 cm and weight was 52 kg. The SF-36 score was PCS 43 and WOMAC score 46, and the evaluation score obtained by summing both and dividing by 2 was 44.5.Based on the interview results, the rehabilitation index was determined to be 5. As a result, a program consisting of 30 sessions (10 sessions × 3 sets) of chair stand-up exercises, each lasting 10 minutes, was selected.

### (2) Update of the rehabilitation program

The selected rehabilitation program was displayed as a video on the smart device's screen to instruct the patient to perform the exercises. Heart rate and calorie consumption were measured at the end of each exercise. Based on the patient's height (average) and weight (overweight), the target heart rate was set at 155 beats per minute, and the target calorie consumption was set at 148 kcal.

At the end of the rehabilitation exercise, the patient's heart rate was 160 beats per minute and calorie consumption was 120 kcal. Since the patient's calorie consumption was within ± 10% of the target value set in the rehabilitation program, it was determined that the prescribed program was appropriate for the patient, and the grade of the rehabilitation program was increased by one level.

While embodiments of the present invention have been provided with reference to the accompanying drawings, the present invention is not limited to these embodiments and may be implemented in various other embodiments within the scope of the invention.

### [Explanation of symbols]

10 PC (personal computer)
21 Smart device 1
22 Smart device 2
23 Smart device 3
33 Wearable sensor device

## Claims

1. A remote medical care support system for selecting and executing a rehabilitation program for an orthopedic patient capable of independent exercise, wherein
the system comprises a smart device and a PC (personal computer) wirelessly connected to the smart device via an Internet connection,
the PC is installed in a medical institution and accessible by a physician or physical therapist and has an application software of the remote medical care support system installed thereon,
the PC is provided with:
means for receiving input of the patient's identification number, profile, and kind of disease treated,
means for transmitting and receiving data to and from the smart device,
a memory device, and
a CPU,
the smart device has the application software of the remote medical support system installed thereon, the smart device is provided with:
means for logging into the remote medical care support system by entering the patient's identification number registered in the PC,
means for receiving input of answers to a plurality of questions in a questionnaire using a health-related quality of life (QOL) assessment scale,
means for transmitting and receiving data to and from the PC, and
means for displaying the rehabilitation program received from the PC on an output screen,
the memory device of the PC stores a plurality of rehabilitation programs in video format corresponding to post-operative conditions of predetermined kinds of diseases,
each rehabilitation program comprises a plurality of exercise programs,
each rehabilitation program has a plurality of grades corresponding to a magnitude of load that is placed on a body of the patient when the exercise program is performed,
each rehabilitation program has a rehabilitation index registered for each grade thereof that is appropriate for the load placed on the body of the patient when the exercise program of that grade is performed,
the system is configured to:
calculate a recovery evaluation score of the patient based on the patient's responses to the plurality of questions in the questionnaire,
determine a rehabilitation index that evaluates the patient's recovery status on a numerical scale based on the calculated recovery evaluation score of the patient,
select a rehabilitation program and its grade to be prescribed to the patient from the plurality of rehabilitation programs stored in the memory device of the PC based on the type of disease of the patient and the rehabilitation index, and
display the selected rehabilitation program and its grade on an output screen of the smart device.

2. The remote medical support system according to Claim 1, the grade of the rehabilitation program selected based on the rehabilitation index is stored in the memory device of the PC together with the identification number of the patient, and by entering the identification number of the patient registered in the PC from the smart device, the grade of the rehabilitation program stored in the memory device of the PC is retrieved and displayed as a video on the output screen of the smart device.

3. The remote medical support system according to Claim 1 or 2, wherein the smart device has health management application software installed in addition to the remote medical care support system application software, and is configured so that the grade of the rehabilitation program selected based on the rehabilitation index is updated based on the patient's exercise biometric information measured using the health management application software.

4. The remote medical support system according to Claim 1 or 2, wherein the smart device is connected wirelessly or via a wired connection to a wearable sensor device, and the grade of the rehabilitation program selected based on the rehabilitation index is updated based on the patient's exercise biometric information measured using the wearable sensor device.

5. The remote medical care support system according to Claim 3 or 4, wherein each grade of each rehabilitation program has registered therein exercise biometric information (exercise target value) that is expected to be measured from a patient executing the exercise instructed by the program of that grade, the exercise target value being set corresponding to the profile of the patient executing the exercise program, and wherein the system can compare exercise biometric information collected from a patient executing the selected grade of rehabilitation program with the exercise target value determined based on the patient's profile to decide whether to change the selected grade of rehabilitation program.

6. The remote medical care support system according to Claim 1 or 2, wherein the predetermined kinds of diseases include musculoskeletal disorders such as low back pain (postoperative lumbar degenerative disease), scoliosis, postoperative knee joint surgery, postoperative hip joint surgery, and chronic low back pain.

7. The remote medical care support system according to Claim 1 or 2, wherein the PC further comprises means for receiving input of the patient's recovery evaluation score calculated based on answers to a plurality of questions in a questionnaire using a health-related QOL evaluation scale, and wherein the system can determine the patient's rehabilitation index based on either the calculated recovery evaluation score or the input recovery evaluation score.

8. A remote medical care support system for selecting and executing a rehabilitation program for an orthopedic patient capable of independent exercise, wherein
the system comprises a smart device and a PC (personal computer) wirelessly connected to the smart device via an internet connection,
the PC is installed in a medical institution and accessible by a physician or physical therapist and has the remote medical care support system application software installed thereon, the PC is provided with:
means for receiving input of patient identification number, profile, and type of treated disease,
means for receiving input of the patient's recovery evaluation score calculated based on answers to a plurality of questions in a questionnaire using a health-related quality of life (QOL) assessment scale,
means for transmitting and receiving data to and from the smart device,
a memory device, and
a CPU,
the smart device has the application software of the remote medical support system installed thereon, the smart device is provided with:
means for logging into the remote medical support system by entering the patient's identification number registered in the PC,
means for transmitting and receiving data with the PC, and
means for displaying the rehabilitation program received from the PC on an output screen as a video,
the memory device of the PC stores a plurality of rehabilitation programs in video format corresponding to post-operative conditions of predetermined kinds of diseases,
each rehabilitation program consists of a plurality of exercise programs,
each rehabilitation program has a plurality of grades corresponding to the magnitude of the load that is placed on the body of the patient when the exercise programs are executed as instructed in the rehabilitation program,
each rehabilitation program has a rehabilitation index registered for each grade thereof that is appropriate for the load placed on the body of the patient when the exercise program of that grade is performed,
the system is configured to:
determine a rehabilitation index that evaluates the patient's recovery status on a numerical scale based on the recovery evaluation score input to the PC,
select a rehabilitation program and a grade thereof from the plurality of rehabilitation programs stored in the memory device of the PC based on the type of disease of the patient and the rehabilitation index, and stores the rehabilitation program and a grade thereof in the memory device of the PC along with the patient's identification number, and
by entering the patient's registered identification number into the smart device, the rehabilitation program stored in the memory device of the PC is retrieved and displayed as a video on the display screen of the smart device.

9. The remote medical care support system according to Claim 8, wherein the PC further comprises means for receiving the identification number of the medical institution prescribing the rehabilitation program together with the identification number of the patient, and the system is configured so that the patient can login to the remote medical care support system by inputting the identification number of the patient and the medical institution registered in the PC into the smart device.

10. The remote medical care support system according to Claim 8 or 9, wherein a password for logging into the remote medical care support system is set in the smart device for the patient, and the patient can log in to the remote medical care support system by inputting the password together with the patient's identification number from the smart device.

11. The remote medical care support system according to Claim 8 or 9, wherein, in addition to the application software of the remote medical care support system, a health management application software is installed on the smart device, and the system is configured such that, based on exercise biometric information of the patient measured using the health management application software, the grade of the rehabilitation program selected based on the rehabilitation index is updated.

12. The remote medical care support system according to Claim 8 or 9, wherein a wearable sensor device is connected to the smart device wirelessly or via cable, and the system is configured such that, based on exercise biometric information of the patient measured using the wearable sensor device, the grade of the rehabilitation program selected based on the rehabilitation index is updated.

13. The remote medical care support system according to Claim 11 or 12, wherein each grade of each rehabilitation program has registered therein exercise biometric information (exercise target value) that is expected to be measured from a patient executing the exercise specified by the program of that grade, the exercise target values being set corresponding to the profile of the patient executing the exercise program, and wherein the system can compare exercise biometric information collected from a patient executing the selected grade of rehabilitation program with the exercise target values determined based on the patient's profile to decide whether to modify the selected grade of rehabilitation program.
